# FASCICULE DE BREVET EUROPEEN

(11) **EP 2 117 522 B1**
(45) Date de publication et mention de la délivrance du brevet: **19.10.2011**
(21) Numéro de dépôt: 08762100.9
(22) Date de dépôt: 15.02.2008
(51) Int. Cl.: A61K 9/70, A61F 13/02

(54) **PATCH POUR APPLICATION CUTANEE**
PFLASTER ZUR ANWENDUNG AUF DER HAUT
PATCH FOR CUTANEOUS APPLICATION

(30) Priorité: 15.02.2007 FR 0753265
(43) Date de publication de la demande: 18.11.2009
(73) Titulaire: DBV Technologies, 92100 Boulogne Billancourt (FR)
(72) Inventeur: DUPONT, Bertrand, F-13100 Aix En Provence (FR)
(74) Mandataire: Becker, Philippe
(86) Numéro de dépôt international: PCT/FR2008/050252
(87) Numéro de publication internationale: WO 2008/104720

(56) Documents cités:
- EP-A2- 1 356 821
- WO-A-02/074286
- WO-A-2005/084255
- US-A- 6 159 497
- US-B1- 6 676 961

## Description

La présente invention concerne de façon générale les dispositifs pour l'application épi- ou trans- cutanée de substances biologiquement actives. L'invention concerne plus particulièrement les patchs ou timbres transdermiques destinés à faciliter l'absorption cutanée de telles substances à des fins de vaccination.

L'épiderme humain constitue une barrière contre l'entrée dans le corps d'agents extérieurs. La peau n'est pas étanche, elle est en fait perméable à un très grand nombre de substances avec un degré de perméabilité variable.

L'absorption percutanée correspond au transfert d'une substance à travers la peau depuis le milieu extérieur jusqu'au sang. Cette absorption est définie comme la somme de deux phénomènes : une pénétration des molécules au sein de la peau entière, suivie d'une résorption par la circulation sanguine ou lymphatique depuis le derme papillaire puis le derme profond. L'étape de pénétration est physiquement une diffusion passive à travers chaque structure du tégument : la couche cornée, l'épiderme de Malpighi, le derme et les annexes cutanées. Une fois absorbée, la substance est distribuée dans l'organisme puis, après avoir été ou non métabolisée, elle est éliminée. Les étapes succédant à l'absorption percutanée sont similaires à celles qui sont rencontrées pour toute autre voie de contamination.

La couche cornée constitue la barrière la plus efficace contre la pénétration d'une substance : anatomiquement, la substance peut pénétrer par deux voies : l'une à travers les espaces intercellulaires de la couche cornée et à travers les cellules cornées elles-mêmes, l'autre par l'intermédiaire des annexes cutanées. Dans le cas d'un vaccin, une fois la couche cornée traversée, la substance est à même de rencontrer les cellules immunologiquement compétentes et, en particulier, les cellules présentatrices d'antigènes comme les cellules de Langerhans dont le rôle est primordial dans la réaction immunologique de l'organisme. Faciliter le passage initial de l'antigène à travers la couche cornée, c'est rendre la vaccination épicutanée plus efficace. Dans le cas d'un patch destiné à l'administration d'une molécule pour un traitement systémique, diminuer temporairement l'efficacité de la couche cornée en tant que barrière, permet d'ouvrir la voie à l'administration transcutanée de principes actifs à forts poids moléculaires et, d'un façon générale, d'améliorer la vitesse d'administration des principes actifs.

La demande EP1356821 concerne l'utilisation successive d'un film adhésif et d'un patch afin de faire pénétrer un vaccin par voie épicutanée. Pour cela, on applique sur la peau un film dont une face est enduite d'un adhésif. L'adhésif présente des propriétés d'adhésion lui permettant de retirer une partie de la couche cornée (ou stratum comeum) de la peau lorsque le film est pelé. Une fois le film pelé, un patch est appliqué sur la partie de la peau venant d'être découverte. Le patch présente une surface adhésive et une surface enduite d'un vaccin. La surface adhésive permet de maintenir le patch sur la peau pendant une durée suffisante pour permettre à l'épiderme d'absorber le vaccin.

Un tel procédé d'administration présente des inconvénients. En effet, l'administration du vaccin nécessite l'application de plusieurs dispositifs en plusieurs étapes. De plus, le positionnement du patch est approximatif par rapport à la zone où le film adhésif a été appliqué. Une bonne absorption du vaccin n'est donc pas garantie. En outre, une étourderie peut conduire à oublier l'application préalable du film adhésif. Ainsi, la quantité de vaccin absorbée par l'épiderme peut être sensiblement réduite. La quantité réellement absorbée par l'épiderme est donc connue de façon relativement imprécise.

L'invention vise à résoudre un ou plusieurs de ces inconvénients. L'invention a ainsi pour objet un patch pour l'application cutanée d'une substance, le patch comprenant une substance destinée à pénétrer l'épiderme, des moyens de mise en contact de la substance avec la peau et une feuille pelable d'exfoliation cutanée permettant une exfoliation de la peau, après mise en place du patch, au niveau de la zone de contact entre ladite substance et la peau.

Selon une variante, le patch comprend :
- un support comprenant au moins une face inférieure présentant une première surface en contact avec ladite substance et une deuxième surface destinée à entrer en contact avec la peau ;
- éventuellement des moyens amovibles maintenant la substance en contact avec ladite première surface;
- la feuille pelable d'exfoliation cutanée étant solidaire du support et comprenant une face inférieure présentant une surface adhésive disposée à l'aplomb de ladite substance ; et
- des moyens pour maintenir en contact la deuxième surface avec la peau lorsque la feuille d'exfoliation a été pelée.

Selon encore une variante, la deuxième surface est adhésive et entoure la première surface.

Selon une autre variante, les moyens amovibles de maintien de la première surface en contact avec la substance comprennent un élément de protection interposé entre la feuille d'exfoliation et le support, l'élément de protection recouvrant la première surface.

Selon encore une autre variante, les moyens pour maintenir en contact la deuxième surface et la substance avec la peau comprennent un adhésif enduisant la deuxième surface, et la deuxième surface présente une partie non recouverte par l'élément de protection.

Selon une variante, le patch comprend une feuille de protection pelable recouvrant et en contact avec ladite partie non recouverte et avec la surface inférieure de la feuille d'exfoliation.

Selon une autre variante, le patch comprend un élément de préhension saillant par rapport au support, l'élément de préhension étant solidaire de la feuille d'exfoliation de façon à la délaminer de la peau lorsqu'il est manipulé.

Selon encore une variante, la partie de préhension fait saillie latéralement par rapport au support, du côté opposé à la partie non recouverte par l'élément de protection.

Selon encore une autre variante, l'élément de préhension est solidaire des moyens amovibles, de sorte que ces moyens amovibles sont retirés lorsque l'élément de préhension est actionné.

Selon une variante, la feuille d'exfoliation et les moyens amovibles sont solidarisés par des premières extrémités respectives.

Selon encore une variante, la partie de préhension est solidaire d'une deuxième extrémité de la feuille d'exfoliation ou des moyens amovibles, cette deuxième extrémité étant opposée à la première.

Selon une autre variante, les moyens amovibles sont formés d'une feuille, et l'élément de préhension est une feuille en contact avec la feuille des moyens amovibles et avec la surface supérieure de la feuille d'exfoliation par l'intermédiaire de faces partiellement enduites d'adhésif.

Selon encore une autre variante, les moyens amovibles et l'élément de préhension sont constitués d'une feuille repliée.

Selon une variante, l'élément de préhension est rabattu et solidarisé de façon amovible sur la surface supérieure du support.

Selon encore une variante, l'élément de préhension fait saillie perpendiculairement à la face inférieure du support.

Selon une autre variante, les moyens amovibles et l'élément de préhension sont formés d'un organe en forme de prisme de section triangulaire dont les bases présentent des renfoncements de préhension, dont une face latérale recouvre la première surface, et dont une autre face latérale est solidarisée à au moins une extrémité de la feuille d'exfoliation.

Selon encore une autre variante, le support et les moyens amovibles maintiennent la substance dans un volume de rétention hermétique.

Selon une variante, la substance présente des (ou est sous forme de) particules, qui sont maintenues au contact de la première surface par des forces électrostatiques de nature coulombienne ou des forces de van der Waals. Dans un mode préféré, la première surface est dotée de propriétés électrostatiques.

Selon une autre variante, le pouvoir adhésif de la surface adhésive de la feuille d'exfoliation est calibré en fonction de la substance.

L'invention porte également sur l'utilisation d'un patch tel que défini ci-dessus pour la fabrication d'une composition pour la délivrance d'une substance à un patient.

Selon une variante, cette utilisation est destinée à la fabrication d'une composition pour la vaccination de sujets, pour la désensibilisation de sujets, ou pour la délivrance de toute substance active à un sujet.

Un autre objet de l'invention concerne l'utilisation d'un patch tel que défini ci-avant pour l'application d'une substance sur la peau, et/ou pour la délivrance d'une substance par voie épi- ou trans-cutanée à un sujet, notamment un mammifère, en particulier un être humain (par exemple enfant ou adulte). Le patch peut notamment être utilisé pour la vaccination de sujets, pour la désensibilisation de sujets, ou pour la délivrance de toute substance active, telle que notamment des (poly)peptides biologiquement actifs et/ou antigéniques, par exemple. L'invention concerne également des méthodes correspondantes, comprenant l'application d'un patch de l'invention sur la peau d'un sujet, et le retrait de la feuille d'exfoliation.

Le patch peut ainsi être utilisé pour la vaccination de sujets contre tout pathogène. Ainsi, un objet particulier de l'invention réside dans une méthode de vaccination d'un sujet contre un pathogène, comprenant (i) l'application d'un patch selon l'invention sur la peau d'un sujet, le patch comprenant un antigène spécifique dudit pathogène, (ii) le retrait de la feuille d'exfoliation et (iii) le maintien du patch pour une période de temps permettant le transfert de l'antigène dans la peau. Le pathogène peut être de nature variée (virus, bactérie, parasite, etc.) et l'antigène est typiquement de nature polypeptidique ou lipidique.

Le patch peut également être utilisé pour la désensibilisation de sujets à des allergènes. Ainsi, un objet particulier de l'invention réside dans une méthode de désensibilisation d'un sujet à un allergène, comprenant (i) l'application d'un patch selon l'invention sur la peau d'un sujet, le patch comprenant un antigène spécifique dudit allergène, (ii) le retrait de la feuille d'exfoliation et (iii) le maintien du patch pour une période de temps permettant le transfert de l'antigène dans la peau.

Le patch peut également être utilisé pour la délivrance de toute substance active. Ainsi, un objet particulier de l'invention réside dans une méthode de délivrance d'une substance active à un sujet, comprenant (i) l'application d'un patch selon l'invention sur la peau d'un sujet, le patch comprenant ladite substance active, (ii) le retrait de la feuille d'exfoliation et (iii) le maintien du patch pour une période de temps permettant le transfert de la substance dans la peau. La substance est typiquement de nature polypeptidique, comme une hormone, une cytokine, un facteur de croissance, un facteur trophique, etc.

La substance contenue dans le patch peut être formulée dans tout véhicule ou excipient adapté, et peut être sous forme solide (poudre), liquide, etc.

D'autres caractéristiques et avantages de l'invention ressortiront clairement de la description qui en est faite ci-après, à titre indicatif et nullement limitatif, en référence aux dessins annexés, dans lesquels :
- la figure 1 est une vue en coupe d'un premier mode de réalisation d'un patch selon l'invention ;
- la figure 2 est une vue de dessus du patch de la figure 1 ;
- la figure 3 est une vue de dessus de chacun des éléments du patch des figures 2 et 3 ;
- la figure 4 est une vue en coupe d'un deuxième mode de réalisation d'un patch selon l'invention ;
- les figures 5 à 8 sont des vues en coupe du patch de la figure 4 durant différentes étapes de sa mise en place sur la peau ;
- la figure 9 est une vue en coupe d'un troisième mode de réalisation d'un patch selon l'invention ;
- la figure 10 est une vue de dessus de chacun des éléments du patch de la figure 9 ;
- la figure 11 est une vue de côté d'un quatrième mode de réalisation d'un patch selon l'invention.

L'invention propose un patch pour l'application cutanée d'une substance, le patch comprenant une feuille pelable d'exfoliation cutanée permettant une exfoliation de la peau, après mise en place du patch, au niveau de la zone de contact entre ladite substance et la peau.

Au sens de l'invention, le terme "exfoliation" ou "abrasion" désigne le retrait d'une partie au moins de la surface de la couche superficielle de la peau, et en particulier d'une partie au moins de la couche cornée (ou stratum corneum) de la peau. A cet égard, la feuille d'exfoliation comporte typiquement une zone adhésive qui entre en contact avec la peau. Lorsqu'elle est retirée (pelée), la feuille d'exfoliation cause une abrasion de la surface de la peau. La feuille d'exfoliation peut par ailleurs comporter, typiquement sur sa zone adhésive qui entre en contact avec la peau, des moyens de micro-perforation, ajoutant ainsi au retrait d'une partie de la surface de la couche cornée une action de micro-perforation de celle-ci, lorsque le dispositif est appliqué. Typiquement, l'exfoliation ainsi pratiquée permet :
- de retirer, sans douleur et à l'endroit où l'administration trans- ou épicutanée aura lieu, la couche superficielle de cellules de la couche cornée, en général mortes, qui constituent la première barrière contre la pénétration des substances, et/ou
- de constituer ainsi une surface nettoyée et dépourvue de poils, sur laquelle le patch peut adhérer sur toute sa surface adhésive, favorisant ainsi l'occlusion et donc le passage de la substance, et/ou
- de créer des micro-perforations dans la couche cornée, par exemple en munissant la feuille d'éléments perforants tels que des micro-aiguilles.

Dans un mode de mise en oeuvre particulier, le patch comprend un support dont une partie de la surface est recouverte de la substance destinée à pénétrer l'épiderme et une autre partie de la surface est destinée à entrer en contact avec la peau. La feuille pelable d'exfoliation cutanée est solidaire de la zone de contact du support et recouvre la surface recouverte de substance. Elle comporte, sur sa face inférieure, une zone adhésive disposée à l'aplomb de la substance. Ainsi, une fois la feuille adhésive pelée, la substance est en contact de la peau à l'emplacement exact où la peau a été exfoliée.

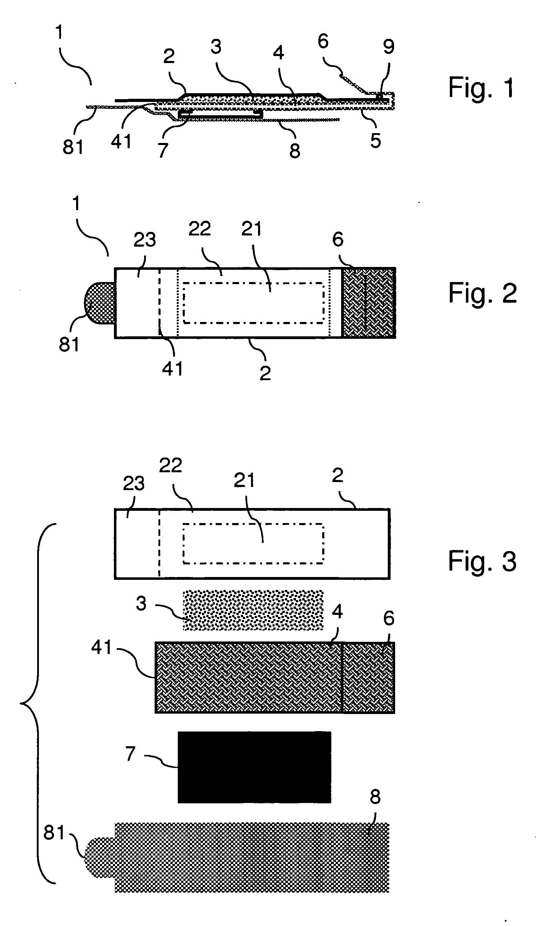

Les figures 1 à 3 illustrent un premier mode de réalisation de l'invention. Le patch 1 comprend un support 2, réalisé par exemple sous forme d'un film. Le support 2 dispose d'une face inférieure présentant une première surface 21 destinée à être maintenue en contact avec une substance 3 et une deuxième surface 22 destinée à être appliquée sur la peau. La substance 3 est destinée à être appliquée sur la peau La surface 21 est située dans un renfoncement du support 2. La surface 22 est adhésive et entoure la surface 21. La surface adhésive 22 permet de maintenir la substance 3 en contact avec la peau durant l'application du patch.

Une feuille 4 recouvre la substance 3 et la maintient en contact avec la surface 21. La feuille 4 forme un volume de rétention de la substance 3 avec la surface 21. Le volume de rétention formé est avantageusement hermétique. De façon connue en soi, la feuille 4 est fixée de façon amovible au support 2. La feuille 4 est repliée au niveau d'une extrémité 41. Un repli 5 s'étend depuis l'extrémité 41 de la feuille 4. Le repli 5 se prolonge en un élément de préhension 6. L'élément de préhension 6 est, replié sur la face supérieure du support 2. L'élément de préhension 6 est maintenu replié de façon amovible par un point d'adhésif 9, de sorte qu'un utilisateur peut rompre cet adhésif pour manipuler l'élément de préhension 6. L'élément 6 est en saillie par rapport au support 2, afin de garantir une manipulation aisée pour l'utilisateur. La feuille 4 ne recouvre pas une partie 23 de la surface adhésive 22. L'utilité de cette partie adhésive 23 sera détaillée par la suite.

Une feuille pelable 7 est solidarisée au repli 5 par tout moyen approprié (collage, soudage...) par son extrémité la plus proche de la partie adhésive 23. Cette feuille pelable 7 est destinée à réaliser une exfoliation cutanée. La feuille 7 est pour cela pourvue d'une face inférieure présentant une surface adhésive. L'exfoliation vise notamment à retirer une partie de la couche cornée afin de favoriser la pénétration transcutanée de la substance 3. Cette surface adhésive est disposée à l'aplomb de la substance 3. Ainsi, la substance 3 sera appliquée exactement à l'emplacement de la peau qui aura été exfolié lors du pelage de la feuille 7.

Une feuille pelable 8 de protection recouvre la partie adhésive 23 et la surface inférieure de la feuille 7, afin d'éviter que des poussières ne viennent se fixer dessus. La feuille pelable 8 peut être réalisée en tout matériau adéquat présentant une faible adhérence pour faciliter son pelage, tel que par exemple en papier ou en polymère (polyester, polyéthylène, éthylène/acétate de vinyle), éventuellement recouvert d'une couche de silicone. La feuille pelable 8 présente en outre une zone de préhension 81, en saillie par rapport à la surface 23 du support 2. Cette zone de préhension 81 permet de faciliter le pelage de la feuille 8 par un utilisateur.

Lorsqu'un utilisateur souhaite appliquer le patch 1, il procède de la façon suivante : il pèle la feuille 8, applique l'adhésif de la feuille exfoliante 7 sur la peau et plaque la surface adhésive 23 sur la peau. La surface adhésive 23 garantit alors un bon positionnement du patch sur la peau. Après avoir rompu le point de colle 9, l'utilisateur exerce une traction sur l'élément de préhension 6, afin d'assurer le pelage de la feuille 7 et le pelage de la feuille 4. Le pelage de la feuille 7 est exercé par un délaminage par rapport à la peau, l'adhésif exfoliant alors la peau avec laquelle il était en contact. Une partie de la peau est ainsi exfoliée et se retrouve à l'aplomb de la substance 3 venant d'être découverte. L'utilisateur n'a plus qu'à plaquer le patch contre la peau avec la paume de la main, pour que la substance 3 entre en contact avec la surface de peau exfoliée.

Le positionnement relatif de la peau exfoliée et de la substance 3 est ainsi particulièrement précis et ne nécessite qu'un nombre réduit de manipulations par l'utilisateur. De plus, la structure du patch garantit que la substance 3 sera bien appliquée sur une peau préalablement exfoliée.

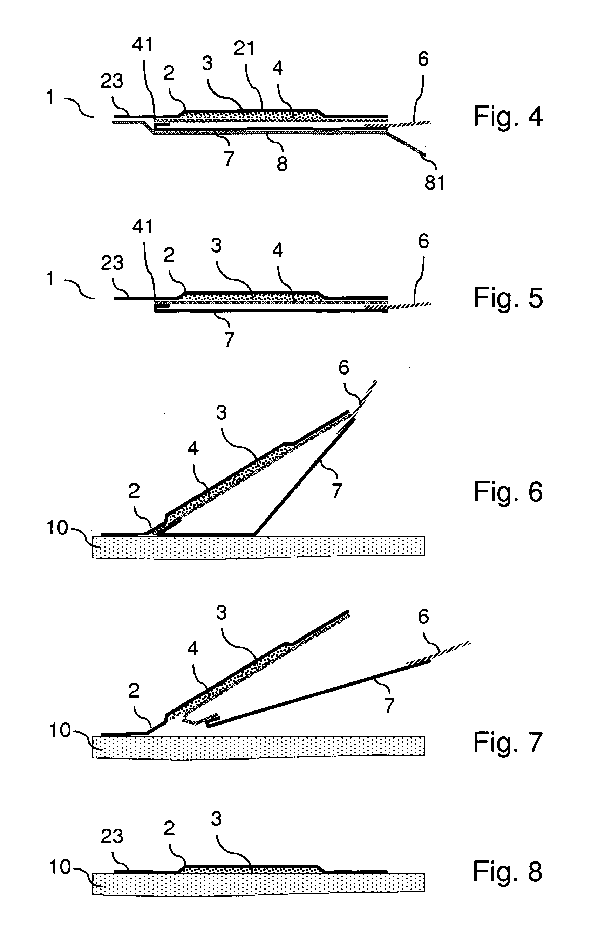

La figure 4 illustre un patch 1 selon un deuxième mode de réalisation. Ce patch 1 comprend un support 2 et une substance 3 similaires à ceux du premier mode de réalisation. Une feuille 4 recouvre la substance 3 et la maintient en contact avec la surface 21. La feuille 4 est pelable et donc fixée de façon amovible au support 2. Comme dans le premier mode de réalisation, la feuille 4 ne recouvre pas la partie adhésive 23 du support 2. La partie adhésive 23 fait ainsi saillie latéralement par rapport à une extrémité 41 de la feuille 4.

Une feuille pelable 7 d'exfoliation cutanée est solidarisée à la feuille 4 par tout moyen approprié (collage, soudage...). Comme dans le premier mode de réalisation, la feuille 7 est pourvue d'une face inférieure présentant une surface adhésive. Cette surface adhésive est disposée à l'aplomb de la substance 3. Une extrémité de la feuille 7 est fixée à l'extrémité 41 de la feuille 4. Un élément de préhension 6 est fixé au niveau de l'extrémité opposée de la feuille 7. L'élément de préhension 6 fait saillie latéralement par rapport au support 2.

Une feuille pelable 8 de protection recouvre la partie adhésive 23 et la surface inférieure de la feuille 7. La feuille pelable 8 présente en outre une zone de préhension 81, en saillie latéralement par rapport au support 2, du même côté que l'élément de préhension.

Les figures 5 à 8 illustrent un procédé d'application du patch 1 sur la peau 10 d'un utilisateur. A la figure 5, l'utilisateur a pelé la feuille 8, pour exposer la partie adhésive 23 et la surface adhésive de la feuille 7. A la figure 6, ces surfaces adhésives ont été mises en contact avec la peau. En exerçant une traction vers le haut sur l'élément de préhension 6, la feuille 7 est délaminée de la peau et exerce son effet exfoliant. A la figure 7, la feuille 7 est intégralement délaminée de la peau. Comme la feuille 7 est solidarisée à l'extrémité 41 de la feuille 4, celle-ci est également entraînée et est pelée par rapport au support 2. La substance 3 est alors découverte et se trouve à l'aplomb de la surface de peau exfoliée. L'utilisateur n'a plus qu'à plaquer le patch 1 contre la peau avec la paume de la main, pour que la substance 3 entre en contact avec la surface de peau exfoliée, comme illustré à la figure 8.

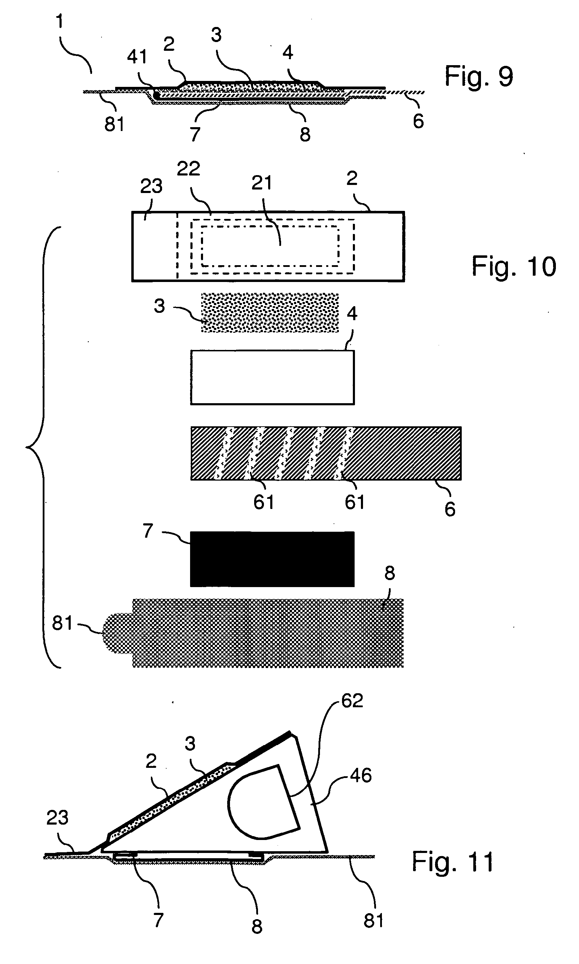

Les figures 9 et 10 illustrent un troisième mode de réalisation d'un patch 1. Ce patch 1 comprend un support 2 et une substance 3 similaires à ceux des modes de réalisation précédents.

Une feuille 4 recouvre la substance 3 et la maintient en contact avec la surface 21. La feuille 4 est pelable et donc fixée de façon amovible au support 2. Comme dans les modes de réalisation précédents, la feuille 4 ne recouvre pas la partie adhésive 23 du support 2. La partie adhésive 23 fait ainsi saillie latéralement par rapport à une extrémité 41 de la feuille 4. La feuille 4 est en contact avec une partie de la surface adhésive 22 sur toute la périphérie de la surface 21. La liaison pelable de la feuille 4 avec le support 2 est assurée par son contact avec la surface adhésive 22. La feuille 4 est également entourée par une autre partie de la surface adhésive 22 qu'elle ne recouvre pas. Cette autre partie est destinée à venir en contact avec la peau et entourer la zone de peau exfoliée.

Une feuille pelable 7 d'exfoliation cutanée a une extrémité fixée à l'extrémité 41 de la feuille 4. Comme dans les modes de réalisation précédents, la feuille 7 est pourvue d'une face inférieure présentant une surface adhésive. Cette surface adhésive est disposée à l'aplomb de la substance 3. Comme illustré à la figure 10, la feuille 7 présente une forme identique à la feuille 4.

Un élément de préhension 6 réalisé sous forme de feuille est intercalé entre les feuilles 4 et 7. L'élément de préhension est ainsi en contact avec les feuilles 4 et 7 par ses deux faces. Cet élément de préhension 6 a une extrémité fixée à l'extrémité 41 de la feuille 4. L'extrémité opposée de l'élément de préhension 6 fait saillie latéralement par rapport au support 2. Les deux faces de l'élément de préhension 6 sont partiellement enduites d'adhésif au niveau de la surface de contact avec les feuilles 4 et 7. Ces faces présentent ainsi une alternance de bandes adhésives 61 avec des zones non enduites d'adhésif. Ces faces partiellement enduites permettent à la fois de garantir une tenue mécanique du patch avant son utilisation, tout en permettant un délaminage de ces faces par rapport aux feuilles 4 et 7. Le pouvoir adhésif doit être suffisamment faible pour permettre un délaminage lorsque l'utilisateur exerce une traction sur l'élément de préhension 6.

Une feuille pelable 8 de protection recouvre la partie adhésive 23 et la surface inférieure de la feuille 7. La feuille pelable 8 présente en outre une zone de préhension 81, en saillie latéralement par rapport à la partie adhésive 23 du support 2.

Lorsqu'un utilisateur souhaite appliquer le patch 1, il procède de la façon suivante : il pèle la feuille 8, applique l'adhésif de la feuille exfoliante 7 sur la peau et plaque la surface adhésive 23 sur la peau. Du fait que des extrémités respectives des feuilles 4 et 7 et de l'élément de préhension 6 soient fixées ensemble, lorsque l'utilisateur exerce une traction sur l'élément de préhension 6, celui-ci entraîne le pelage simultané des feuilles 4 et 7. Une partie de la peau est ainsi exfoliée et se retrouve à l'aplomb de la substance 3 qui vient d'être découverte. L'utilisateur n'a plus qu'à plaquer le patch contre la peau avec la paume de la main, pour que la substance 3 entre en contact avec la surface de peau exfoliée.

Par ailleurs, lors du pelage des feuilles 4 et 7, la surface inférieure adhésive de la feuille 7 vient en contact avec la surface de la feuille 4 qui maintient la substance dans le volume de rétention. Ainsi, d'éventuels résidus de la substance sur la feuille 4 sont neutralisés.

La figure 11 illustre un quatrième mode de réalisation d'un patch 1. Ce patch 1 comprend un support 2 et une substance 3 similaires à ceux des modes de réalisation précédents. Un applicateur 46 est utilisé comme moyen amovible pour retenir la substance 3 en contact avec la surface 21 avant son application cutanée. De plus l'applicateur 46 est également utilisé comme élément de préhension. L'applicateur 46 se présente ainsi sous forme d'un solide en saillie perpendiculairement par rapport à la face inférieure du support 2. L'applicateur 46 présente globalement une forme de prisme de section triangulaire, dont les bases sont avantageusement munies de renfoncements de préhension 62 pour les doigts de l'utilisateur. Une face latérale du prisme recouvre la surface 21 et maintient la substance 3 en contact avec cette surface 21. Cette face latérale présentera avantageusement une faible adhérence pour pouvoir être aisément séparée du support 2. Cette face latérale ne recouvre pas la partie 23 de la surface 22.

Une feuille pelable 7 d'exfoliation cutanée a une extrémité fixée à une autre face latérale du prisme. L'autre extrémité de la feuille 7 est liée de façon amovible ou sécable à cette autre surface du prisme. Comme dans les modes de réalisation précédents, la feuille 7 est pourvue d'une face inférieure présentant une surface adhésive. Cette surface adhésive est disposée à l'aplomb de la substance 3. Une feuille de protection pelable 8 recouvre la partie 23 et la surface inférieure de la feuille 7.

Lorsqu'un utilisateur souhaite appliquer le patch 1, il procède de la façon suivante : il retire la feuille de protection pelable 8, puis applique la partie 23 et la surface inférieure de la feuille 7 contre la peau. En soulevant la partie arrière de l'applicateur 46 (à l'opposée de la partie 23 du support 2), on rompt la liaison sécable d'une extrémité de la feuille 7 avec l'applicateur 46. En exerçant une traction sur l'applicateur 46, on découvre la substance 3 et on délamine la feuille 7 de la peau. L'applicateur pèle en effet la feuille 7 en entraînant l'extrémité avant à laquelle il est fixé. En passant la paume de la main sur la face supérieure du support, on applique la substance 3 contre la peau venant d'être exfoliée.

On peut également envisager de disposer une feuille de protection entre l'applicateur 46 et le support 2, afin de maintenir la substance 3 contre la surface 21. Une telle feuille de protection peut être réalisée sous forme de film siliconé. Cette feuille de protection peut être montée pelable sur le support 2 et présenter une extrémité avant fixée à l'applicateur 46. Ainsi, lors d'une traction de l'applicateur 46, cette feuille de protection sera pelée.

Dans ces différents modes de réalisation, on peut prévoir que la surface 21 présente des propriétés électrostatiques. On peut alors prévoir qu'une substance 3 sous forme de particules soit maintenue en contact avec la surface 21 par des forces électrostatiques.

Du fait de l'association de la substance 3 et d'une feuille exfoliante 7 à l'aplomb de cette substance, on peut avantageusement calibrer le pouvoir adhésif de la feuille 7 en fonction de la substance 3 ou de son dosage.

L'invention concerne également l'utilisation d'un patch tel que défini précédemment pour la délivrance de molécules à l'organisme. Le Patch peut notamment être utilisé pour la vaccination de sujets, pour la désensibilisation de sujets, ou pour la délivrance de toute substance active. Les substances actives contenues dans le patch peuvent être de toute nature, de préférence polypeptidique, et peuvent être des composés biologiquement actifs ou des antigènes, par exemple.

## Revendications

1. Patch pour l'application cutanée d'une substance, **caractérisé en ce qu'**il comprend :
- une substance (3) destinée à pénétrer l'épiderme,
- un support (2) destiné à être appliqué sur la peau, une partie de la surface du support étant recouverte de la substance et une autre étant destinée à entrer en contact avec la peau, et
- une feuille pelable (7) fixée de façon amovible à la partie du support destinée à entrer en contact avec la peau, ladite feuille couvrant la partie du support recouverte de ladite substance et présentant sur sa face externe une zone adhésive,
l'application du patch sur la peau causant l'adhésion de la feuille pelable, le retrait de ladite feuille permettant une exfoliation de la peau au niveau de la zone d'application et la mise en contact de ladite substance avec la peau au niveau de ladite zone exfoliée.

2. Patch selon la revendication 1, **caractérisé en ce qu'**il comprend :
- un support (2) comprenant au moins une face inférieure présentant une première surface (21) en contact avec ladite substance (3) et une deuxième surface (22, 23) destinée à entrer en contact avec la peau ;
- éventuellement des moyens amovibles (4) maintenant la substance (3) en contact avec ladite première surface (21) ;
- la feuille pelable (7) d'exfoliation cutanée étant solidaire du support (2) et comprenant une face inférieure présentant une surface adhésive disposée à l'aplomb de ladite substance (3) ; et
- des moyens pour maintenir en contact la deuxième surface (22, 23) avec la peau lorsque la feuille d'exfoliation (7) a été pelée.

3. Patch pour application cutanée selon la revendication 2, dans lequel la deuxième surface (22) est adhésive et entoure la première surface (21).

4. Patch pour application cutanée selon la revendication 2 ou 3, dans lequel les moyens amovibles (4) de maintien de la première surface (21) en contact avec la substance (3) comprennent un élément de protection interposé entre la feuille (7) d'exfoliation et le support (2), l'élément de protection recouvrant la première surface (21).

5. Patch pour application cutanée selon la revendication 4, dans lequel les moyens pour maintenir en contact la deuxième surface et la substance avec la peau comprennent un adhésif enduisant la deuxième surface (22, 23), et dans lequel la deuxième surface présente une partie non recouverte (23) par l'élément de protection.

6. Patch pour application cutanée selon la revendication 5, comprenant une feuille de protection pelable (8) recouvrant et en contact avec ladite partie non recouverte (23) et avec la surface inférieure de la feuille d'exfoliation (7).

7. Patch pour application cutanée selon l'une quelconque des revendications 2 à 6, comprenant un élément de préhension (6) saillant par rapport au support (2), l'élément de préhension (6) étant solidaire de la feuille d'exfoliation (7) de façon à la délaminer de la peau lorsqu'il est manipulé.

8. Patch pour application cutanée selon les revendications 5 et 7, dans lequel la partie de préhension (6) fait saillie latéralement par rapport au support (2), du côté opposé à la partie non recouverte (23) par l'élément de protection.

9. Patch pour application cutanée selon la revendication 7 ou 8, dans lequel l'élément de préhension (6) est solidaire des moyens amovibles (4), de sorte que ces moyens amovibles (4) sont retirés lorsque l'élément de préhension (6) est actionné.

10. Patch pour application cutanée selon la revendication 9, dans lequel la feuille d'exfoliation (7) et les moyens amovibles (4) sont solidarisés par des premières extrémités (41) respectives.

11. Patch pour application cutanée selon la revendication 10, dans lequel la partie de préhension (6) est solidaire d'une deuxième extrémité de la feuille d'exfoliation (7) ou des moyens amovibles (4), cette deuxième extrémité étant opposée à la première.

12. Patch pour application cutanée selon la revendication 10, dans lequel les moyens amovibles (4) sont formés d'une feuille, et dans lequel l'élément de préhension (6) est une feuille en contact avec la feuille des moyens amovibles (4) et avec la surface supérieure de la feuille d'exfoliation (7) par l'intermédiaire de faces partiellement enduites d'adhésif (61).

13. Patch pour application cutanée selon l'une quelconque des revendications 7 à 12, dans lequel les moyens amovibles (4) et l'élément de préhension (6) sont constitués d'une feuille repliée.

14. Patch pour application cutanée selon l'une quelconque des revendications 7 à 13, dans lequel l'élément de préhension (6) est rabattu et solidarisé de façon amovible (9) sur la surface supérieure du support (2).

15. Patch pour application cutanée selon l'une quelconque des revendications 2 à 10, dans lequel l'élément de préhension (6) fait saillie perpendiculairement à la face inférieure du support (2).

16. Patch pour application cutanée selon la revendication 15, dans lequel les moyens amovibles et l'élément de préhension sont formés d'un organe (46) en forme de prisme de section triangulaire dont les bases présentent des renfoncements de préhension (62), dont une face latérale recouvre la première surface (21), et dont une autre face latérale est solidarisée à au moins une extrémité de la feuille d'exfoliation (7).

17. Patch pour application cutanée selon l'une quelconque des revendications 2 à 16, dans lequel le support (2) et les moyens amovibles (4) maintiennent la substance dans un volume de rétention hermétique.

18. Patch pour application cutanée selon l'une quelconque des revendications 2 à 17, dans lequel la première surface (21) est dotée de propriétés électrostatiques ou non et dans lequel la substance présente des particules maintenues au contact de la surface par des forces électrostatiques de nature coulombienne ou des forces de van der Waals.

19. Patch pour application cutanée selon l'une quelconque des revendications 2 à 18, dans lequel le pouvoir adhésif de la surface adhésive de la feuille d'exfoliation est calibré en fonction de la substance.

20. Utilisation d'un patch tel que défini dans l'une quelconque des revendications précédentes pour la fabrication d'une composition pour la délivrance d'une substance à un patient.

21. Utilisation selon la revendication 20, pour la vaccination de sujets, pour la désensibilisation de sujets, ou pour la délivrance de toute substance active à un sujet.

## Claims

1. A patch for cutaneous application of a substance, wherein said patch comprises:
- a substance (3) intended to penetrate the epidermis,
- a support (2) intended to be applied to the skin, wherein a part of the surface of the support is covered by the substance and another part is intended to make contact with the skin, and
- a peelable film (7) fixed removably to the part of the support intended to make contact with the skin, said film covering the part of the support covered by said substance and presenting on its external face an adhesive zone,
the application of the patch to the skin causing the adhesion of the peelable film, the removal of said film allowing exfoliation of the skin at the level of the application zone and making contact of said substance with the skin at the level of said exfoliated zone.

2. The patch as claimed in claim 1, wherein said patch comprises:
- a support (2) comprising at least one lower face having a first surface (21) in contact with said substance (3) and a second surface (22, 23) intended to make contact with the skin;
- optionally removable means (4) keeping the substance (3) in contact with said first surface (21);
- the cutaneous peelable exfoliation film (7) being fixed to the support (2) and comprising a lower face having an adhesive surface placed in front of said substance (3); and
- means for keeping the second surface (22, 23) in contact with the skin when the exfoliation film (7) has been peeled.

3. The patch for cutaneous application as claimed in claim 2, wherein the second surface (22) is adhesive and encloses the first surface (21).

4. The patch for cutaneous application as claimed in claim 2 or 3, wherein the removable means (4) for keeping the first surface (21) in contact with the substance (3) comprise a protective element interposed between the exfoliation film (7) and the support (2), the protective element covering the first surface (21).

5. The patch for cutaneous application as claimed in claim 4, wherein the means for keeping the second surface and the substance in contact with the skin comprise an adhesive coating the second surface (22, 23), and wherein the second surface has a portion non-covered (23) by the protective element.

6. The patch for cutaneous application as claimed in claim 5, comprising a peelable protective film (8) covering and in contact with said non-covered part (23) and with the lower face of the exfoliation film (7).

7. The patch for cutaneous application as claimed in any one of claims 2 to 6, comprising a prehension element (6) projecting relative to the support (2), the prehension element (6) being fixed to the exfoliation film (7) so as to delaminate it from the skin when it is handled.

8. The patch for cutaneous application as claimed in claims 5 and 7, wherein the prehension part (6) projects laterally relative to the support (2), to the side opposite the non-covered part (23) by the protective element.

9. The patch for cutaneous application as claimed in claim 7 or 8, wherein the prehension element (6) is fixed to the removable means (4), such that these removable means (4) are withdrawn when the prehension element (6) is actuated.

10. The patch for cutaneous application as claimed in claim 9, wherein the exfoliation film (7) and the removable means (4) are fixed by respective first ends (41).

11. The patch for cutaneous application as claimed in claim 10, wherein the prehension part (6) is fixed to a second end of the exfoliation film (7) or the removable means (4), this second end being opposite the first.

12. The patch for cutaneous application as claimed in claim 10, wherein the removable means (4) are formed by a film, and wherein the prehension element (6) is a film in contact with the film of the removable means (4) and with the upper surface of the exfoliation film (7) by means of faces partially coated with adhesive (61).

13. The patch for cutaneous application as claimed in any one of claims 7 to 12, wherein the removable means (4) and the prehension element (6) are constituted by a folded film.

14. The patch for cutaneous application as claimed in any one of claims 7 to 13, wherein the prehension element (6) is folded back and fixed removably (9) on the upper surface of the support (2).

15. The patch for cutaneous application as claimed in any one of claims 2 to 10, wherein the prehension element (6) projects perpendicularly to the lower face of the support (2).

16. The patch for cutaneous application as claimed in claim 15, wherein the removable means and the prehension element are formed by an element (46) in the form of a prism of triangular cross-section whereof the bases have prehension indents (62), whereof a lateral face covers the first surface (21), and whereof another lateral face is fixed to at least one end of the exfoliation film (7).

17. The patch for cutaneous application as claimed in any one of claims 2 to 16, wherein the support (2) and the removable means (4) keep the substance in a volume of hermetic retention.

18. The patch for cutaneous application as claimed in any one of claims 2 to 17, wherein the first surface (21) is endowed with electrostatic properties or not and wherein the substance has particles kept in contact with the surface by electrostatic forces of the type of Coulomb or van der Waals forces.

19. The patch for cutaneous application as claimed in any one of claims 2 to 18, wherein the adhesive power of the adhesive surface of the exfoliation film is calibrated as a function of the substance.

20. Use of a patch such as defined in any one of the preceding claims for the manufacture of a composition for delivering a substance to a patient.

21. Use as claimed in claim 20, for the vaccination of subjects, for the desensitisation of subjects, or for the delivery of any active substance to a subject.

## Patentansprüche

1. Pflaster für die Applikation einer Substanz auf der Haut, **dadurch gekennzeichnet, dass** es umfasst:
- eine Substanz (3), die die Epidermis penetrieren soll,
- einen Träger (2), der auf der Haut appliziert werden soll, wobei ein Teil der Trägeroberfläche mit der Substanz beschichtet ist und ein anderer Teil mit der Haut in Kontakt kommen soll, und
- einen abziehbaren Film (7), der in ablösbarer Weise an dem Teil des Trägers befestigt ist, der mit der Haut in Kontakt kommen soll, wobei besagter Film den Teil des Trägers abdeckt, der mit besagter Substanz beschichtet ist, und auf seiner Außenseite eine adhäsive Zone aufweist, wobei die Applikation des Pflasters auf der Haut die Adhäsion des abziehbaren Films bedingt, wobei das Abziehen besagten Films eine Exfoliation der Haut im Bereich der Applikationszone und das Inkontaktbringen besagter Substanz mit der Haut im Bereich besagte abgeschilferter Zone erlaubt.

2. Pflaster gemäß Anspruch 1, **dadurch gekennzeichnet, dass** es umfasst:
- einen Träger (2), umfassend wenigstens eine Unterseite, die eine erste Oberfläche (21) in Kontakt mit besagter Substanz (3) und eine zweite Oberfläche (22, 23) aufweist, die mit der Haut in Kontakt kommen soll;
- gegebenenfalls ablösbare Mittel (4), die die Substanz (3) in Kontakt mit besagter erster Oberfläche (21) halten;
- wobei der abziehbare Film (7) für eine Exfoliation der Haut mit dem Träger (2) verbunden ist und eine Unterseite umfasst, die eine senkrecht zu besagter Substanz (3) angeordnete adhäsive Oberfläche aufweist; und
- Mittel zur Aufrechterhaltung des Kontaktes der zweiten Oberfläche (22, 23) mit der Haut, wenn der Exfoliationsfilm (7) abgezogen worden ist.

3. Pflaster zur Applikation auf der Haut gemäß Anspruch 2, bei dem die zweite Oberfläche (22) adhäsiv ist und die erste Oberfläche (21) umschließt.

4. Pflaster zur Applikation auf der Haut gemäß Anspruch 2 oder 3, bei dem die ablösbaren Mittel (4) zur Aufrechterhaltung des Kontaktes der ersten Oberfläche (21) mit der Substanz (3) ein Schutzelement zwischen dem Exfoliationsfilm (7) und dem Träger (2) umfassen, wobei das Schutzelement die erste Oberfläche (21) abdeckt.

5. Pflaster zur Applikation auf der Haut gemäß Anspruch 4, bei dem die Mittel zur Aufrechterhaltung des Kontaktes der zweiten Oberfläche und der Substanz mit der Haut ein Adhäsiv umfassen, mit dem die zweite Oberfläche (22, 23) beschichtet ist, und bei dem die zweite Oberfläche einen von dem Schutzelement nicht abgedeckten Teil (23) aufweist.

6. Pflaster zur Applikation auf der Haut gemäß Anspruch 5, umfassend einen abziehbaren Schutzfilm (8), der mit besagtem nicht abgedecktem Teil (23) und mit der Unterseite des Exfoliationsfilms (7) in Kontakt steht und abdeckt.

7. Pflaster für die Applikation auf der Haut gemäß einem der Ansprüche 2 bis 6, umfassend ein Greifelement (6), das in Bezug auf den Träger (2) herausragt, wobei das Greifelement (6) mit dem Exfoliationsfilm (7) verbunden ist, so dass er von der Haut abgelöst wird, wenn das Greifelement (6) gehandhabt wird.

8. Pflaster zur Applikation auf der Haut gemäß Ansprüchen 5 und 7, bei dem das Greifteil (6) in Bezug auf den Träger (2) auf der entgegengesetzten Seite des von dem Schutzelement nicht bedeckten Teils (23) seitlich herausragt.

9. Pflaster zur Applikation auf der Haut gemäß Anspruch 7 oder 8, bei dem das Greifelement (6) mit den ablösbaren Mitteln (4) verbunden ist, so dass diese ablösbaren Mittel (4) abgezogen werden, wenn das Greifelement (6) betätigt wird.

10. Pflaster zur Applikation auf der Haut gemäß Anspruch 9, bei dem der Exfoliationsfilm (7) und die ablösbaren Mittel (7) über die jeweiligen ersten Enden (41) verbunden sind.

11. Pflaster zur Applikation auf der Haut gemäß Anspruch 10, bei dem das Greifteil (6) mit einem zweiten Ende des Exfoliationsfilms (7) oder den ablösbaren Mitteln (4) verbunden ist, wobei dieses zweite Ende dem ersten Ende gegenüberliegt.

12. Pflaster zur Applikation auf der Haut gemäß Anspruch 10, bei dem die ablösbaren Mittel (4) als ein Film ausgebildet sind und bei dem das Greifelement (6) ein Film ist, der mit dem Film der ablösbaren Mittel (4) und mit der Oberseite des Exfoliationsfilms (7) mithilfe von Oberflächen in Kontakt steht, die partiell mit Adhäsiv beschichtet sind (61).

13. Pflaster zur Applikation auf der Haut gemäß einem der Ansprüche 7 bis 12, bei dem die ablösbaren Mittel (4) und das Greifelement (6) aus einem gefalteten Film gebildet sind.

14. Pflaster zur Applikation auf der Haut gemäß einem der Ansprüche 7 bis 13, bei dem das Greifelement (6) auf die Oberseite des Trägers (2) umgeschlagen ist und auf ablösbare Weise (9) verbunden ist.

15. Pflaster zur Applikation auf der Haut gemäß einem der Ansprüche 2 bis 10, bei dem das Greifelement (6) senkrecht zur Unterseite des Trägers (2) herausragt.

16. Pflaster zur Applikation auf der Haut gemäß Anspruch 15, bei dem die ablösbaren Mittel und das Greifelement aus einem Element (46) in Form eines Prismas mit dreieckigem Querschnitt gebildet sind, dessen Grundflächen Greifvertiefungen (62) aufweisen, dessen eine Seitenfläche die erste Oberfläche (21) abdeckt und dessen andere Seitenfläche mit wenigstens einem Ende des Exfoliationsfilms (7) verbunden ist.

17. Pflaster zur Applikation auf der Haut gemäß einem der Ansprüche 2 bis 16, bei dem der Trägers (2) und die ablösbaren Mittel (4) die Substanz in einem hermetisch verschlossenen Retentionsvolumen haltern.

18. Pflaster für die Applikation auf der Haut gemäß einem der Ansprüche 2 bis 17, bei dem die erste Oberfläche (21) mit elektrostatischen Eigenschaften ausgestattet sein kann und bei dem die Substanz Partikel aufweist, die durch elektrostatische Kräfte, wie die Coulomb Kraft oder die van der Waals Kräfte, mit der Oberfläche in Kontakt gehalten werden.

19. Pflaster zur Applikation auf der Haut gemäß einem der Ansprüche 2 bis 18, bei dem die Adhäsionskraft der adhäsiven Oberfläche des Exfoliationsfilms abhängig von der Substanz kalibriert ist.

20. Verwendung eines Pflasters wie in einem der vorhergehenden Ansprüche definiert, zur Herstellung einer Zusammensetzung für die Abgabe einer Substanz an einen Patienten.

21. Verwendung gemäß Anspruch 20, für die Impfung von Personen, für die Desensibilisierung von Personen oder für die Abgabe einer beliebigen aktiven Substanz an eine Person.
